(19) Europäisches Patentamt
European Patent Office
Office européen des brevets

(11) EP 0 565 975 B1

(12) **EUROPÄISCHE PATENTSCHRIFT**

(45) Veröffentlichungstag und Bekanntmachung des Hinweises auf die Patenterteilung: **04.09.1996 Patentblatt 1996/36**

(51) Int. Cl.[6]: **C07C 45/62**, C07C 49/04, C07D 317/12, C07D 319/06, C07C 43/303, C07C 41/48, C07C 31/125, C07C 29/17

(21) Anmeldenummer: **93105548.7**

(22) Anmeldetag: **03.04.1993**

(54) **Verfahren zur Herstellung von Isoprenderivaten**

Process for the preparation of isoprenoid derivatives

Procédé pour la préparation de dérivés isopréniques

(84) Benannte Vertragsstaaten:
**AT BE CH DE DK ES FR GB IT LI NL**

(30) Priorität: **16.04.1992 CH 1270/92**

(43) Veröffentlichungstag der Anmeldung:
**20.10.1993 Patentblatt 1993/42**

(73) Patentinhaber: **F. HOFFMANN-LA ROCHE AG**
**4002 Basel (CH)**

(72) Erfinder:
- **Broger, Emil Albin**
**CH-4312 Magden (CH)**
- **Müller, Robert-Karl**
**CH-4058 Basel (CH)**

(74) Vertreter: **Cottong, Norbert A. et al**
**F.Hoffmann-La Roche AG**
**Patent Department (PLP),**
**124 Grenzacherstrasse**
**4070 Basel (CH)**

(56) Entgegenhaltungen:

| | |
|---|---|
| **EP-A- 0 034 804** | **EP-A- 0 039 830** |
| **EP-A- 0 397 042** | **EP-A- 0 398 132** |
| **EP-A- 0 409 530** | **WO-A-92/16536** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 565 975 B1

Printed by Rank Xerox (UK) Business Services
2.13.4/3.4

## Beschreibung

Die vorliegende Erfindung betrifft ein neues Verfahren zur Herstellung von Isoprenderivaten der allgemeinen Formel

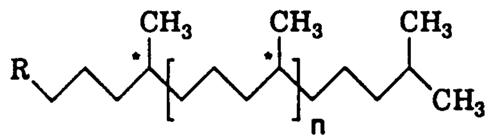

I

worin die Asymmetriezentren unabhängig voneinander die (R)- oder (S)-Konfiguration aufweisen können, R einen Rest der Formeln

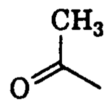
(a)

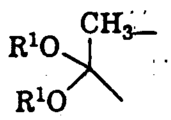
(b)

oder

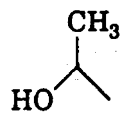
(c)

darstellt, $R^1$ niederes Alkyl oder beide $R^1$ zusammen Aethylen oder Propylen bedeuten und n für die Zahl 0, 1 oder 2 steht, welches dadurch gekennzeichnet ist, dass man eine in der (E)- oder (Z)-Form vorliegende Verbindung der allgemeinen Formel

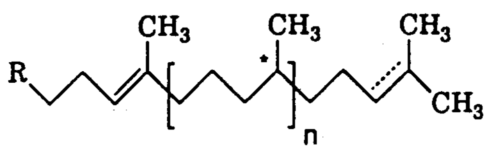

II

worin für R, n und ein gegebenenfalls vorhandenes Asymmetriezentrum obige Bedeutungen stehen, mit einem Ruthenium-Komplex eines optisch aktiven atropisomeren Diphosphins asymmetrisch hydriert.

Die Verbindungen der obigen Formel I sind bekannte oder zum Teil auch neue Verbindungen und geeignete Zwischenprodukte in der Synthese von z.B. (R,R,R)-$\alpha$-Tocopherol, Vitamin $K_1$ oder auch Phytol und dergleichen.

Die als Ausgangsmaterial verwendeten Verbindungen der Formel II sind zum grössten Teil bekannte Verbindungen. Die allenfalls noch neuen Verbindungen können leicht in zur Herstellung der bekannten Verbin dungen analoger Weise hergestellt werden. Das Ausgangsmaterial der Formel II kann gegebenenfalls an der bezeichneten Stellung eine weitere Doppelbindung enthalten, die dann unter den Bedingungen der asymme trischen Hydrierung ebenfalls hydriert wird. Die katalytische Hydrierung solcher Verbindungen in Gegenwart von Pd als Katalysators ist in EP-A-0 034 804 beschrieben. In EP-A-0 398 132 wird die enantioselektive Hydrierung von Allylalkoholen in Gegenwart von Ru-Komplexen mit optisch aktiven Phosphin-Liganden beschrieben.

Als Ruthenium-Komplexe von atropisomeren Diphosphinen kommen, im Rahmen der vorliegenden Erfindung, insbesondere in Frage Verbin dungen der allgemeinen Formeln

$$[RuL]^{2+}(Z)_2, \quad Ru(Z^1)_2L \quad \text{oder} \quad [Ru(Z^2)_{2-m}(L)(X)](Z^3)_m$$

III IV V

worin Z $BF_4^{\ominus}$, $ClO_4^{\ominus}$, $B(Phenyl)_4^{\ominus}$ oder $PF_6^{\ominus}$ darstellt, $Z^1$ Halogen oder die Gruppe Y-$COO^{\ominus}$ oder Y-$SO_3^{\ominus}$ bedeutet, Y niederes Alkyl, Phenyl, halogeniertes niederes Alkyl oder halogeniertes Phenyl bedeutet, $Z^2$ Halogen, X Benzol, Hexamethylbenzol oder p-Cymol und m die Zahl 1 oder 2 darstellt, $Z^3$ Halogen, $BF_4^{\ominus}$, $ClO_4^{\ominus}$ oder $B(Phenyl)_4^{\ominus}$ bedeutet und L einen optisch aktiven, atropisomeren Diphosphin-Liganden bedeutet.

Als optisch aktive atropisomere Diphosphin-Liganden kommen, im Rahmen der vorliegenden Erfindung, insbesondere in Frage Verbindungen der allgemeinen Formel in der (R) oder (S) Form

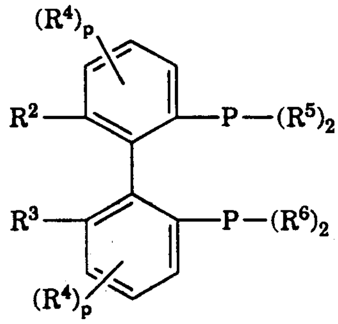

VI

worin $R^2$ und $R^3$ unabhängig voneinander niederes Alkyl, niederes Alkoxy, Hydroxy, geschütztes Hydroxy oder $R^2$ und $R^3$ zusammen $-O-CH_2-O-CH_2-O-$ bedeuten, $R^4$ für niederes Alkyl oder niederes Alkoxy und p für die Zahl 0, 1 oder 2 stehen und $R^5$ und $R^6$ unabhängig voneinander Aryl, einen fünfgliedrigen Heteroaromaten oder zusammen mit dem Phosphoratom eine Gruppe der Formel

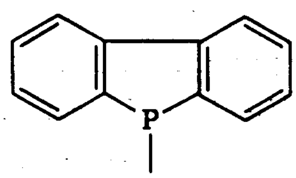

bedeuten.

Der Ausdruck "niederes Alkyl" bedeutet, im Rahmen der vorliegenden Erfindung, geradkettige oder verzweigte Alkylgruppen mit 1 bis 4 Kohlenstoffatomen wie Methyl, Aethyl, Propyl, Isopropyl, n-Butyl, Isobutyl und tert.Butyl. Der Ausdruck "niederes Alkoxy" bedeutet Gruppen, in denen der Alkylrest die vorhergehende Bedeutung hat. Als Schutzgruppen für die Hydroxygruppen kommen, im Rahmen der vorliegenden Erfindung, insbesondere in Frage die üblichen Aether-bildenden Gruppen, wie z.B. Benzyl, Allyl, Benzyloxymethyl, niederes Alkoxymethyl oder auch 2-Methoxyäthoxymethyl und dergleichen. Der Ausdruck "fünfgliedriger Heteroaromat" steht, im Rahmen der vorliegenden Erfindung, für einen Substituenten der Formel

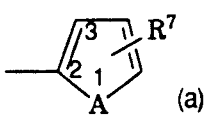 (a) 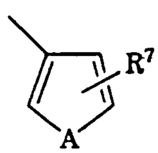 (b) 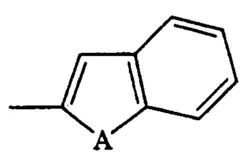 (c) 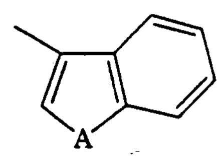 (d)

In den Substituenten der Formeln (a) bis (d) wiederum bedeutet A Sauerstoff, Schwefel oder $-NR^8$. Der Substituent $R^7$ bedeutet hier Wasserstoff, niederes Alkyl, insbesondere Methyl, oder niederes Alkoxy, insbesondere Methoxy, und $R^8$ steht für niederes Alkyl, vorzugsweise Methyl.

Der Ausdruck "Halogen" bedeutet im Rahmen der vorliegenden Erfindung Fluor, Chlor, Brom oder Jod.

Der Ausdruck "halogeniertes niederes Alkyl" bedeutet im Rahmen der vorliegenden Erfindung niedere Alkylgruppen mit einer variablen Anzahl von Halogenatomen, insbesondere Chlor oder Fluor, wobei sich vorzugsweise wenigstens ein Halogenatom in $\alpha$-Stellung zur -COO- Gruppe befindet.

Bevorzugte halogenierte niedere Alkylgruppen sind perfluorierte und perchlorierte niedere Alkylgruppen, beispielsweise Trifluormethyl, Pentafluoräthyl und dergleichen.

Der Ausdruck "Aryl" bedeutet im Rahmen der vorliegenden Erfindung insbesondere den Phenylrest, welcher sowohl unsubstituiert als auch in ortho, meta oder para-Stellung oder auch mehrfach substituiert sein kann. Als Substituenten kommen hier in Frage Phenyl, niedere Alkyl- oder niedere Alkoxygruppen, vorzugsweise Methyl- oder Methoxygruppen, oder auch Di-niederes Alkylamino, vorzugsweise Dimethylaminogruppen, sowie Fluor, Trialkylsilyl, wie Trimethylsilyl oder auch Sulfamoyl wie z.B. N,N-Dimethylaminosulfamoyl und dergleichen. Der Ausdruck kann zudem auch Naphthyl bedeuten. Der Ausdruck "halogeniertes Phenyl" bedeutet vorzugsweise Perfluorphenyl oder Perfluorbiphenyl.

Das Zeichen (*) bedeutet, dass es sich bei dem in Frage stehenden C-Atom um ein asymmetrisches C-Atom handelt.

Die erfindungsgemass verwendeten Diphosphin-Liganden der Formel VI sind bekannte Verbindungen oder Analoge bekannter Verbindungen, welche leicht in zur Herstellung der bekannten Verbindungen analoger Weise hergestellt werden können.

Die Komplexe der Formeln IV und V sind ebenfalls bekannte Verbindungen oder Analoge bekannter Verbindungen, welche leicht in zur Herstellung der bekannten Verbindungen analoger Weise hergestellt werden können, beispielsweise gemäss EP 397 042.

Die Komplexe der Formel III sind ebenfalls bekannte Verbindungen oder Analoge bekannter Verbindungen und können leicht in an sich bekannter Weise hergestellt werden, beispielsweise gemäss Takaya et al., J. Org. Chem. 1987, 52, 3174-3177.

Die erfindungsgemässen, asymmetrischen Hydrierungen erfolgen zweckmässig in einem unter den Reaktionsbedingungen inerten, organischen Lösungsmittel. Als derartige Lösungsmittel können insbesondere genannt werden, niedere Alkohole wie z.B. Methanol, Aethanol oder Trifluoräthanol, oder Gemische derartiger Alkohole mit halogenierten Kohlenwasserstoffen wie Methylenchlorid, Chloroform, Hexafluorbenzol und dergleichen, oder mit Aethern wie Diäthyläther, Tetrahydrofuran oder Dioxan, und dergleichen. Ferner können hier genannt werden Ketone wie Aceton, Diäthylketon, Methyläthylketon usw., Ester wie Essigsäuremethylester, Carbonsäuren wie Ameisensäure, Essigsäure und dergleichen, oder auch Wasser.

Das Verhältnis von Ruthenium zu Ligand L in den Komplexen der Formeln III, IV und V liegt zweckmässig zwischen etwa 0,5 und etwa 2 Mol, vorzugsweise bei etwa 1 Mol Ruthenium pro Mol Ligand. Das Verhältnis von Ruthenium zu den zu hydrierenden Substanzen liegt zweckmässig zwischen etwa 0,01 und etwa 1 Mol %, vorzugsweise zwischen etwa 0,05 und etwa 0,5 Mol %.

Die asymmetrische Hydrierung mit den Komplexen der Formeln III, IV und V erfolgt zweckmässig bei einer Temperatur von etwa 0°C bis etwa 100°C. Diese Hydrierung erfolgt zweckmässig auch unter Druck, vorzugsweise bei einem Druck von etwa 5 bis etwa 200 bar, besonders bevorzugt von etwa 30 bis etwa 100 bar.

In Analogie zur Verwendung der optisch aktiven, atropisomeren Liganden der Formel VI, können ebenfalls auch optisch aktive Verbindungen des Binaphthyl-Typus der folgenden allgemeinen Formel

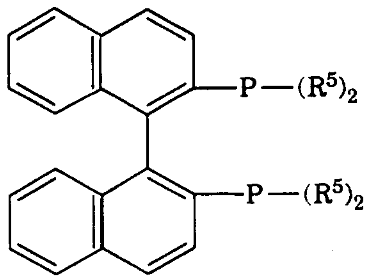

VII

worin $R^5$ und $R^6$ obige Bedeutung haben, verwendet werden. Die Binaphthylringe können hierbei in üblicher Weise substituiert sein.

Die folgenden Beispiele dienen zur Illustrierung der Erfindung und stellen in keine Weise irgendeine Beschränkung dar. In diesen Beispielen haben die gewählten Abkürzungen folgende Bedeutung:

| | |
|---|---|
| GC = | Kapillar-Gaschromatographie |
| e.e. = | Enantiomeric Excess |
| DC = | Dünnschichtchromatographie |
| RT = | Raumtemperatur |
| OAc = | Acetyl |
| TFA = | Trifluoracetyl |

THF = Tetrahydrofuran

Alle Temperaturen sind in °Celsius angegeben.

2-Furyl-BIPHEMP = (6,6-Dimethylbiphenyl-2,2'-diyl)bis(di-2-furylphosphin)

2-Furyl-MeOBIPHEP = (6,6'-Dimethoxybiphenyl-2,2'-diyl)bis(di-2-furylphosphin)

MeOBIPHEP = (6,6'-Dimethoxybiphenyl-2,2'-diyl)bis(diphenylphosphin)

3,5-TMS-MeOBIPHEP = (6,6'-Dimethoxybiphenyl-2,2'-diyl)bis{bis-[3,5-di(trimethylsilyl)phenyl]phosphin}

(3,4,5-MeO)-MeOBIPHEP = (6,6'-Dimethoxybiphenyl-2,2'-diyl)bis[bis-(3,4,5-trimethoxyphenyl)phosphin]

p-DMAS-MeOBIPHEP = (6,6'-Dimethoxybiphenyl-2,2'-diyl)bis{bis-[p-(N,N-dimethylaminosulfamoyl)phenyl]-phosphin}

2-Thienyl$_2$BIPHEMP = P,P-Diphenyl-P',P'-di-2-thienyl-(6,6'-dimethylbiphenyl-2,2'-diyl)diphosphin

BIPHEMP-DIPHOL = 5,5'-(6,6'-Dimethylbiphenyl-2,2'-diyl)-di-5H-benzo[b]phosphindol

HO/IpropO-BIPHEP = (6-Isopropoxy-6'-hydroxybiphenyl-2,2'-diyl)bis(diphenylphosphin)

IpropO-BIPHEP = (6,6'-Diisopropoxybiphenyl-2,2'-diyl)bis(diphenylphosphin)

p-Biphenyl--MeOBIPHEP = (6,6'-Dimethoxybiphenyl-2,2'-diyl)bis(di-4-biphenylylphosphin)

BnO-BIPHEP = (6,6'-Dibenzyloxybiphenyl-2,2'-diyl)bis(diphenylphosphin)

p-DMAPh-MeOBIPHEP = (6,6'-Dimethoxybiphenyl-2,2'-diyl)bis{bis-[p-(N,N-dimethylamino)phenyl]phosphin}

p-Anisyl-MeOBIPHEP = (6,6'-Dimethoxybiphenyl-2,2'-diyl)bis[di-(p-methoxyphenyl)phosphin]

2-Thienyl-BIPHEMP = (6,6'-Dimethylbiphenyl-2,2'-diyl)bis(di-2-thienylphosphin)

2-Thienyl-MeOBIPHEP = (6,6'-Dimethoxybiphenyl-2,2'-diyl)bis(di-2-thienylphosphin)

TriMeO-BIPHEP = (4,4',5,5',6,6'-Hexamethoxybiphenyl-2,2'-diyl)bis(diphenylphosphin)

BIPHOMP = (5,7-Dihydro-dibenz[c,e]oxepin-1,11-diyl)bis(diphenylphosphin)

p-Tolyl-MeOBIPHEP = (6,6'-Dimethoxybiphenyl-2,2'-diyl)bis[di-p-tolylphosphin]

3-Furyl-MeOBIPHEP = (6,6'-Dimethoxybiphenyl-2,2'-diyl)bis(di-3-furylphosphin)

Benzofuryl-MeOBIPHEP = (6,6'-methoxybiphenyl-2,2'-diyl)-bis-[di-(2-benzo[b]furanyl)phosphin]

MeFuryl-MeOBIPHEP = (6,6'-Dimethoxybiphenyl-2,2'-diyl)bis[bis(5-methylfuran-2-yl)phosphin]

Beispiel 1

a) In einer Glove-Box (< 1 ppm Sauerstoff) wurden 8,33 mg (0,0255 mMol) Di($\eta^2$-acetato)-($\eta^4$-cycloocta-1,5-dien)ruthenium(II) hergestellt gemäss B.Heiser et al., Tetrahedron: Asymmetry 2, 51 (1991) und 13,8 mg (0,0255 mMol) (R)-(6,6'-Dimethylbiphenyl-2,2'-diyl)bis(di-2-furylphosphin) in 1 ml Methylenchlorid und 1 ml Methanol gelöst und die orange Lösung 18 Stunden bei Raumtemperatur gerührt. Nach Zugabe von 8,9 mg (0,051 mMol) 50-proz. wässriger $HBF_4$ in 0,5 ml Methanol wurde die gelbe Katalysatorlösung 5 Minuten gerührt.

b) In einer Glove-Box wurde der Glaseinsatz eines 30 ml-Autoklaven mit 1,00 g (5,09 mMol) (Z)-6,10-Dimethylundec-5-en-2-on, 8 ml Methanol und der gemäss a) hergestellten Katalysatorlösung beladen. Die Hydrierung wurde

bei 25° und 60 bar Wasserstoff während 18 Stunden durchgeführt. Die Hydrierlösung wurde am Rotationsverdampfer bei 20°/20 mbar eingedampft, der Rückstand in Aether gelöst und die Lösung zur Abtrennung des Katalysators durch ein Kieselgelpolster filtriert. Das Eluat wurde wie oben eingedampft. Der Rückstand (0,96 g gelbes Oel) bestand gemäss Kapillar-GC aus 8,6% Edukt, 72,9% (R)-6,10-Dimethylundecan-2-on und 13,6% des entsprechenden Dimethylacetals. Der e.e. des Produktes betrug 91,4%. Die e.e.-Bestimmung wurde nach der von A. Knierzinger et al. (Helv. Chim. Acta 1990, 73, 1087) beschriebenen Methode durchgeführt.

Beispiel 2

a) In einer Glove-Box (< 1 ppm Sauerstoff) wurden 6,1 mg (0,0188 mMol) Di($\eta^2$-acetato)-($\eta^4$-cycloocta-1,5-dien)ruthenium(II) und 10,2 mg (0,0188 mMol) (R)-(6,6'-Dimethoxybiphenyl-2,2'-diyl)bis(di-2-furylphosphin) in 1 ml Methylenchlorid und 1 ml Methanol gelöst und die orange Lösung 18 Stunden bei Raumtemperatur gerührt. Nach Zugabe von 6,6 mg (0,0375 mMol) 50-proz. wässriger $HBF_4$ in 0,5 ml Methanol wurde die gelbe Katalysatorlösung 5 Minuten gerührt.

b) In einer Glove-Box wurde der Glaseinsatz eines 30 ml-Autoklaven mit 1,00 g (3,75 mMol) (R,Z)-6,10,14-Trimethylpentadec-5-en-2-on (95,8% e.e.), 8 ml Methanol und der gemäss a) hergestellten Katalysatorlösung beladen. Die Hydrierung wurde bei 25° und 60 bar Wasserstoff während 24 Stunden durchgeführt. Die Hydrierlösung wurde am Rotationsverdampfer bei 20°/20 mbar eingedampft und der Rückstand (0,99 g gelbes Oel) an 40 g Kieselgel chromatographiert; n-Hexan-Aether 5:1 eluierten 873 mg farbloses Oel, das gemäss Kapillar-GC aus 0,6% Edukt und 99,2% 6,10,14-Trimethylpentadecan-2-on bestand. Der Gehalt an (R,R)-6,10-14-Trimethylpentadecan-2-on betrug 93,9% gemäss der von A. Knierzinger et al. (Helv. Chim. Acta 1990, 73, 1087) beschriebenen Methode zur Bestimmung der 4 möglichen Isomeren (entspricht 91,2% e.e. bezüglich Asymmetriezentrum C-6).

Beispiel 3

Eine Lösung von 20,4 mg (0,0255 mMol) $Ru(OAc)_2$[(S)-MeOBIPHEP] (hergestellt gemäss B. Heiser et al., Tetrahedron: Asymmetry 2, 51 (1991)) und 8,94 mg (0,0509 mMol) 50-proz. wässriger $HBF_4$ in 4 ml Methanol wurde 1 Stunde gerührt. Nach Zugabe von 1,01 g (5,09 mMol) rac.(E)-6,10-Dimethylundec-5-en-2-ol und weiteren 5 ml Methanol wurde die Hydrierung bei 25° und 35 bar während 18 Stunden durchgeführt. Die Hydrierlösung wurde am Rotationsverdampfer bei 20°/20 mbar eingedampff, der Rückstand (0,95 g gelbes Oel) in Aether gelöst und die Lösung durch ein Kieselgelpolster filtriert. Das Eluat wurde wie oben eingedampß. Der Rückstand (670 mg gelbes Oel) bestand gemäss Kapillar-GC aus 16,8% Edukt und 83,2% 6,10-Dimethylundecan-2-ol.

Zur e.e.-Bestimmung wurden 200 mg des Alkohols mit 0,3 g Pyridiniumchlorochromat in Methylenchlorid bei 25° während 2 Stunden oxidiert. Aus der braunen Reaktionslösung wurden nach Filtration durch Kieselgel 0,19 g eines farblosen Oels erhalten, das gemäss GC 75,9% (R)-6,10-Dimethylundecan-2-ol von 74,1% e.e. enthielt.

Beispiel 4

Eine Lösung von 20,4 mg (0,0255 mMol) $Ru(OAc)_2$[(S)-MeOBIPHEP] und 5,33 mg (0,028 mMol) $HBF_4$-Diäthyläther-Komplex (Aldrich) in 4 ml Methanol wurde 1 Stunde gerührt. Nach Zugabe von 1,23 g (5,09 mMol) (E)-6,10-Dimethyl-2,2-Dimethoxy-5-undecen und weiteren 5 ml Methanol wurde die Hydrierung bei 25° und 35 bar während 18 Stunden durchgeführt. Die Hydrierlösung wurde am Rotationsverdampfer bei 20°/20 mbar eingedampß, der Rückstand (0,98 g gelbes Oel) in Aether gelöst und die Lösung durch ein Kieselgelpolster filtriert. Das Eluat wurde wie oben eingedampft. Der Rückstand (790 mg gelbes Oel) bestand gemäss Kapillar-GC aus 51% Edukt und 37% (R)-6,10-Dimethyl-2,2-dimethoxyundecan von 70,4% e.e. Der e.e.-Wert wurde in der für das entsprechende Keton in Beispiel 1 beschriebenen Weise durchgeführt.

Beispiele 5-21

In zu Beispiel 1 analoger Weise wurden weitere Hydrierungen durchgeführt und die Resultate in Tabelle 1 zusammengefasst.

**Tabelle 1.** Hydrierung von (E)- oder (Z)-6,10-Dimethylundec-2-en-5-on mit [Ru(MeOBIPHEP)](Z)$_2$ als Katalysator [a)]

| Bsp. | Edukt | Z | MeOBIPHEP Konfig. | S/C b) | Lösungsmittel (ml) | P bar | T °C | Umsatz % c) | Selektivität % d) | e.e. % e) | Konfig. |
|---|---|---|---|---|---|---|---|---|---|---|---|
| 5 | E | $BF_4$ | S | 200 | MeOH (10) | 35 | 25 | 93 | 95 | 77,0 | R |
| 6 | E | $BF_4$ | S | 200 | MeOH (10) | 60 | 25 | 93 | 93 | 80.4 | R |
| 7 | Z | $ClO_4$ f) | S | 200 | MeOH (9), $CH_2Cl_2$ (1) | 60 | 25 | 65 | 95 | 73,1 | S |
| 8 | E | $BF_4$ | S | 200 | MeOH (9), $CH_2Cl_2$ (1) | 60 | 25 | 94 | 94 | 78,7 | R |
| 9 | Z | $BF_4$ | R | 200 | MeOH (0,5), $CHCl_3$ (9,5) | 60 | 25 | 82 | 98 | 74,4 | R |
| 10 | Z | $BF_4$ | R | 200 | MeOH (0,5), AcOH (9,5) | 60 | 25 | 33 | 79 | 75,0 | R |
| 11 | Z | $BF_4$ | R | 200 | MeOH (0,5), Aceton (9,5) | 60 | 25 | 69 | 97 | 73,3 | R |
| 12 | E | $BF_4$ | S | 200 | MeOH (0,5), THF (9,5) | 60 | 25 | 97 | 98 | 81,2 | R |
| 13 | E | $BF_4$ | S | 200 | MeOH (0,5), $Et_2O$ (9,5) | 60 | 25 | 95 | 97 | 88,7 | R |
| 14 | Z | $BF_4$ | S | 200 | MeOH (0,5), $Et_2O$ (9,5) | 60 | 25 | 96 | 97 | 87,0 | S |
| 15 | Z | $BF_4$ | R | 200 | MeOH (0,5), AcOEt (9,5) | 60 | 25 | 56 | 97 | 85,0 | R |
| 16 | E | $BF_4$ | S | 200 | MeOH (0,5), THF (2,5), $Et_2O$ (7) | 60 | 25 | 96 | 97 | 86,6 | R |

Tabelle 1 (Forts.)

| Bsp. | Edukt | X | MeOBIPHEP Konfig. | S/C b) | Lösungsmittel (ml) | P bar | T °C | Umsatz % c) | Selektivität % d) | e.e. % e) | Konfig. |
|---|---|---|---|---|---|---|---|---|---|---|---|
| 17 | Z | $BF_4$ | R | 200 | MeOH (0,5), $C_6F_6$ (9,5) | 35 | 25 | 96 | 97 | 88,6 | R |
| 18 | E | $BF_4$ | R | 2000 | $CF_3CH_2OH$ (10) | 60 | 25 | 100 | 97 | 69,0 | S |
| 19 | Z | $BF_4$ | R | 200 | $CF_3CH_2OH$ (0,5), $Et_2O$ (9,5) | 60 | 25 | 100 | 98 | 86,6 | R |
| 20 | Z | $BF_4$ | R | 200 | $(CF_3)_2CHOH$ (2), $Et_2O$ (8) | 60 | 25 | 94 | 97 | 82,4 | R |
| 21 | Z | $BF_4$ | S | 200 | $CH_2Cl_2$ (0,5), $Et_2O$ (9,5) | 60 | 80 | 92 | 93 | 77,4 | S |

a) 1 g (E)- oder (Z)-Keton in 10 ml Lösungsmittel, Reaktionszeit 18 h.

b) Molares Verhältnis Edukt/Katalysator

c) Gemäss GC, 25m SE 54-Säule.

d) In alkoholischen Lösungsmitteln lag das Produkt zum Teil (<20%) als Ketal vor. Selektivität = Ausbeute Produkte (Keton + Ketal) bezüglich umgesetztes Edukt.

e) GC-Bestimmung, s. Bsp. 1.

f) Hergestellt analog Bsp. 1a) mit 70-proz. $HClO_4$.

MeOH = Methanol; AcOH = Essigsäure; $Et_2O$ = Diäthyläther, AcOEt = Aethylacetat; $C_6F_6$ = Hexafluorbenzol.

Beispiele 22-30

In zu Beispiel 1 analoger Weise wurden weitere Hydrierungen durchgeführt und die Resultate in Tabelle 2 zusammengefasst.

**Tabelle 2.** Asymmetrische Hydrierung von (E)- oder (Z)-6,10-Dimethylundec-5-en-2-on in Gegenwart von $Ru(Z^1)_2$[(R)-2-FurylMeOBIPHEP] bzw. {Ru[(R)-2-FurylMeOBIPHEP]}$(Z)_2$ a)

| Bsp. | Edukt | Z | Lösungsmittel (ml) | P bar | T °C | t h | Umsatz % b) | Selektivität % b) | e.e. % b) | Konfig. |
|---|---|---|---|---|---|---|---|---|---|---|
| 22 | E | $BF_4$ | MeOH (9), $CH_2Cl_2$ (1) | 35 | 25 | 18 | 64 | 99 | 90,0 | S |
| 23 | E | $BF_4$ | MeOH (9), $CH_2Cl_2$ (1) | 60 | 25 | 18 | 84 | 99 | 91,5 | S |
| 24 | Z | $BF_4$ | MeOH (9), $CH_2Cl_2$ (1) | 60 | 25 | 18 | 90 | 97 | 91,4 | R |
| 25 | E | $BF_4$ | MeOH (9), $CH_2Cl_2$ (1) | 60 | 80 | 6 | 100 | 86 | 84,4 | S |
| 26 | Z | $BF_4$ | MeOH (9), $CH_2Cl_2$ (1) | 60 | 80 | 6 | 100 | 89 | 89,2 | R |
| 27 | Z | $BF_4$ | MeOH (0,5), $Et_2O$ (9,5) | 60 | 80 | 6 | 100 | 95 | 89,8 | R |
| 28 | Z | $BF_4$ | MeOH (0,5), $Et_2O$ (9,5) | 60 | 100 | 6 | 95 | 92 | 83,0 | R |
| 29 | Z | $BF_4$ | MeOH (9), $CH_2Cl_2$ (1) | 60 | 0 | 142 | 48 | 93 | 93,4 | R |
| 30 | Z | $Z^1$ TFA c) | MeOH (9), $CH_2Cl_2$ (1) | 60 | 25 | 18 | 35 | 78 | 80,1 | R |

a) 1 g Edukt in 10 ml Lösung; S/C 200.
b) s. Tab. 1
c) $Ru(TFA)_2$[(R)-2-FurylMeOBIPHEP] wurde in zu Beispiel 1 analoger Weise aus $[Ru(TFA)_2COD]_2$ [B. Heiser et al., Tetrahedron: Asymmetry 2, 51 (1991)] und (R)-2-FurylMeOBiPHEP hergestellt.

Beispiele 31-49

In zu Beispiel 1 analoger Weise wurden weitere Hydrierungen durchgeführt und die Resultate in Tabelle 3 zusammengefasst.

Tabelle 3

| Asymmetrische Hydrierung von (E)- oder (Z)-6,10-Dimethylundec-5-en-2-on mit $[RuL]^{2+}(Z)_2$ als Katalysator a) | | | | | | |
|---|---|---|---|---|---|---|
| Bsp. | Edukt | Chiraler Ligand | Umsatz % a) | Selektivität % a) | e.e. % a) | Konfig. |
| 31 | Z | (S)-(3,5-TMS)-MeOBIPHEP | 100 | 16 b | 87,6 | S |
| 32 | Z | (S)-(3,4,5-MeO)-MeOBIPHEP | 100 | 71 b) | 75,4 | S |
| 33 | E | (R)-p-DMAS-MeOBIPHEP | 18 | 75 b) | 84,2 | S |
| 34 | E | (R)-(2-Thienyl)$_2$BIPHEMP | 76 | 85 | 70,0 | S |
| 35 | E | (R)-BIPHEMP-DIPHOL | 54 | 89 | 83,1 | S |
| 36 | E | (R)-HO/IpropO-BIPHEP | 100 | 88 | 76,7 | S |
| 37 | E | (R)-IpropO-BIPHEP | 100 | 89 | 73,6 | S |
| 38 | E | (R)-p-BnO-BIPHEP | 89 | 95 | 78,4 | S |
| 39 | E | (R)-p-Biphenyl-MeOBIPHEP | 66 | 92 | 76,2 | S |
| 40 | E | (S)-p-DMAPh-MeOBIPHEP | 100 | 86 | 70,6 | R |
| 41 | E | (R)-Anisyl-MeOBIPHEP | 100 | 73 | 74,6 | S |
| 42 | Z | (S)-2-Thienyl-BIPHEP | 99 | 42 b) | 80,0 | S |
| 43 | Z | (S)-2-Thienyl-BIPHEMP | 98 | 43 b | 73,6 | S |
| 44 | E | (S)-TriMeO-BIPHEP | 100 | 91 | 78,7 | R |
| 45 | E | (S)-BIPHOMP | 98 | 92 | 81,2 | R |
| 46 | E | (S)-p-Tolyl-MeOBIPHEP | 100 | 90 | 72,1 | R |
| 47 | E | (S)-3-Furyl-MeOBIPHEP | 95 | 92 | 74,8 | R |
| 48 | E | (R)-Benzofuryl-MeOBIPHEP | 45 | 98 | 93,3 | S |
| 49 | Z | (R)-MeFuryl-MeOBIPHEP | 97 | 95 | 92,0 | R |

a) s. Tab. 1.
b) Tiefere Selektivität wegen Ueberhydrierung zum entsprechenden Alkohol.

Beispiel 50

Das gemäss den Beispielen 1 und 2 verwendete (R)-(6,6'-Dimethoxybiphenyl-2,2'-diyl)bis(di-2-furylphosphin) wurde wie folgt hergestellt:

a) Zu einer aus 1,5 ml 2-Jodfuran (67% rein) und 0,30 g (12,3 mMol) Magnesiumspänen in 10 ml Tetrahydrofuran hergestellten Grignardlösung wurde bei Raumtemperatur innerhalb von 15 Minuten eine Lösung von 0,30 g (0,442 mMol) (R)-(6,6'-Dimethoxybiphenyl-2,2'-diyl)bis(phosphonsäurediphenylester) in 10 ml Tetrahydrofuran getropft. Nach beendeter Zugabe wurde 1 Stunde auf 40° erhitzt. Zur Aufarbeitung wurde mit 50 ml ges. $NH_4Cl$-Lösung und 50 ml Essigsäureäthylester versetzt, die Phasen getrennt, und die organische Phase wurde mit ges. NaCl-Lösung gewaschen, über $MgSO_4$ getrocknet, filtriert und eingeengt. Der Rückstand wurde in der minimalen Menge $CH_2Cl_2$ gelöst und die Lösung auf eine Säule von 50 g Kieselgel aufgetragen. Elution mit Essigsäureäthylester und dann mit Tetrahydrofuran lieferte 0,20 g eines Feststoffes, welcher aus 10 ml tert. Butylmethyläther umkristallisiert wurde. Man erhielt 0,16 g (R)-(6,6'-Dimethoxybiphenyl-2,2'-diyl)bis(di-2-furylphosphinoxid) als gelbliche Kristalle, Smp. 272° (Thermoanalyse); $[\alpha]^{20}_{D}$ = +89,6 (c = 1,0, $CHCl_3$).

b) Ein 0,5 l Vierhalssulfierkolben, versehen mit Kühler, Thermometer, Tropftrichter und mechanischem Rührer, wurde unter Argon mit 2,90 g (5,0 mMol) (R)-(6,6'-Dimethoxybiphenyl-2,2'-diyl)bis(di-2-furylphosphinoxid), 10 ml (41,9 mMol) Tributylamin, 60 ml Xylol-Isomerengemisch und 4,0 ml (5,37 g, 39,6 mMol) Trichlorsilan beschickt. Die

milchig-weisse Mischung wurde 4 Stunden unter Rückfluss gekocht, wobei eine beinahe durchscheinende Lösung entstand. Nach Abkühlung wurden unter gutem Rühren 100 ml deoxygenierte 30% Natronlauge so zugetropft, dass die Innentemperatur 70° nicht überstieg, und die Mischung wurde noch 1 Stunde bei 70° gerührt. Nach Zugabe von $H_2O$ und $CH_2Cl_2$ wurden die Phasen getrennt, und die organische Phase wurde mit 2 x 50 ml 30% Natronlauge, $H_2O$, ges. $NH_4Cl$- und ges. NaCl-Lösung gewaschen, über $MgSO_4$ getrocknet, filtriert und eingedampft. Das erhaltene weisse Pulver (4,40 g) wurde in $CH_2Cl_2$ gelöst, die Lösung mit Aethanol versetzt, und das $CH_2Cl_2$ wurde am Rotationsverdampfer abgedampft. Der ausgefallene Festkörper wurde abfiltriert, mit Aethanol und Pentan gewaschen und am Hochvakuum (~ 10 Pa) während 1 Stunde bei 100° getrocknet. Man erhielt 2,50 g (R)-(6,6'-Dimethoxybiphenyl-2,2'-diyl)bis(di-2-furylphosphin) als gelbliche Kristalle; Smp.176°; $[\alpha]_D^{20}$ = +6,9 (c = 1, $CHCl_3$).

Der als Ausgangsmaterial verwendete (R)-(6,6'-Dimethoxybiphenyl-2,2'-diyl)bis(phosphonsäure-diphenylester) bzw. die entsprechende (S)-Verbindung wurden wie folgt hergestellt:

a) In einem 1 l Vierhalssulfierkolben, versehen mit Kühler, Thermometer, Rührer und Aufsatz für Inertgasbehandlung, wurden unter Argon 76,5 g (0,122 Mol) (2-Jod-3-methoxyphenyl)phosphonsäure-diphenylester (75% rein) und 25,0 g (0,393 Mol) aktiviertes Kupferpulver vorgelegt und 200 ml N,N-Dimethylformamid zufliessen gelassen. Die dunkelbraune Suspension wurde 1 Stunde auf 140° erhitzt (Oelbadtemperatur), wonach gemäss DC-Analyse vollständiger Umsatz eingetreten war. Das abgekühlte Reaktionsgut wurde mit etwas Methylenchlorid in einen Rundkolben transferiert und am Rotationsverdampfer bei 70° zur Trockene eingedampft. Der Rückstand wurde mit 200 ml Methylenchlorid versetzt, die Mischung gut durchgerührt und filtriert, und der Filterrückstand mit 100 ml Methylenchlorid gewaschen. Das Filtrat wurde dreimal mit 100 ml ges. $NH_4Cl$-Lösung gewaschen, wobei bei der ersten Waschoperation von wenig gebildetem Festkörper filtriert wurde, und anschliessend über $MgSO_4$ getrocknet, filtriert und eingeengt. Nach Trocknung am Hochvakuum (~ 10 Pa) während 2 Stunden bei 80° erhielt man 59,6 g rohen (RS)-(6,6'-Dimethoxybiphenyl-2,2'-diyl)bis(phosphonsäure-diphenylester).

ba) In einem 1 l Rundkolben wurde eine Lösung von 59,6 g des rohen, gemäss a) erhaltenen Diphenylesters in 50 ml Dichlormethan vorgelegt und mit einer Lösung von 35,8 g (0,10 Mol) (-)-O,O'-Dibenzoyl-L-weinsäure in 100 ml Essigsäureäthylester versetzt. Die Lösung wurde dann am Rotationsverdampfer bei 600 mbar eingedampft, wobei das $CH_2Cl_2$ abdestillierte und ein weisser Festkörper ausfiel. Dieser wurde abgenutscht, gewaschen mit dreimal 20 ml Essigsäureäthylester und 20 ml Hexan und am Hochvakuum (~ 10 Pa) getrocknet. Man erhielt 21,8 g (R)-Diphenylester/(-)-DBW-Addukt als weisses Pulver. $[\alpha]_D^{20}$ = -95,6 (c = 1 in Aethanol).

Die Mutterlaugen und Waschlösungen wurden zur Gewinnung des anderen Enantiomeren beiseite gestellt.

bb) Das gemäss ba) erhaltene Material wurde in einem 1 l Erlenmeyer mit Magnetrührer mit 100 ml Dichlormethan, 50 ml ges. $NaHCO_3$-Lösung und 50 ml entionisiertem Wasser verrührt, bis aller Festkörper in Lösung gegangen war (30 Minuten). Die Phasen wurden getrennt, und die organische Phase wurde zweimal mit 100 ml halbges. $NaHCO_3$-Lösung, 50 ml entionisiertem Wasser und 50 ml ges. NaCl-Lösung gewaschen, über $MgSO_4$ getrocknet, filtriert und eingedampft. Der ölige Rückstand wurde mit 20 ml tert.-Butylmethyläther versetzt, wobei Kristallisation eintrat. Nach Eindampfen und Trocknen am Hochvakuum (~ 10 Pa) während 1 Stunde bei 60° erhielt man 13,8 g (R)-(6,6'-Dimethoxybiphenyl-2,2'-diyl)bis(phosphonsäure-diphenylester) als weisse Kristalle. Smp. 125-125,5°; $[\alpha]_D^{20}$ = -18,9 (c = 1 in $CHCl_3$).

ca) In einem 1 l Rundkolben wurden die Mutterlaugen und Waschlösungen aus ba) eingedampft. Der Rückstand wurde in 100 ml Dichlormethan aufgenommen und die Lösung während 30 Minuten mit 50 ml ges. $NaHCO_3$-Lösung und 50 ml entionisiertem Wasser verrührt. Die Phasen wurden getrennt, und die organische Phase wurde mit 100 ml halbges. $NaHCO_3$-Lösung, 50 ml entionisiertem Wasser und 50 ml ges. NaCl-Lösung gewaschen, über $MgSO_4$ getrocknet, filtriert und eingeengt. Das erhaltene braune Oel wurde in 50 ml Dichlormethan aufgenommen, und die Lösung wurde mit einer Lösung von 18,0 g (0,050 Mol) (+)-O,O'-Dibenzoyl-D-weinsäure in 100 ml Essigsäureäthylester versetzt. Die Lösung wurde dann am Rotationsverdampfer bei 600 mbar aulkonzentriert, wobei das $CH_2Cl_2$ abdestillierte und ein weisser Festkörper ausfiel. Dieser wurde abgenutscht, dreimal mit 20 ml Essigsäureäthylester und 20 ml Hexan gewaschen und am Hochvakuum (~ 10 Pa) getrocknet. Man erhielt 22 g (S)-Diphenylester/(+)-DBW-Addukt als leicht gelbliches Pulver. $[\alpha]_D^{20}$ = +96 (c = 1 in Aethanol).

cb) Das gemäss ca) erhaltene Material wurde wie in bb) beschrieben aufgearbeitet. Man erhielt 13,9 g (S)-(6,6'-Dimethoxybiphenyl-2,2'-diyl)bis(phosphonsäure-diphenylester) als weisse Kristalle. Smp. 124-125°; $[\alpha]_D^{20}$ = +18,7 (c = 1 in $CHCl_3$).

d) Der als Ausgangsmaterial verwendete (2-Jod-3-methoxyphenyl)phosphonsäure-diphenylester wurde wie folgt hergestellt:

daa) In einem 0,5 l Vierhalskolben, versehen mit Rührer, Kühler, Thermometer und Aufsatz für Inertgasbehandlung, wurde unter Argon eine Aufschlämmung von 13,0 g (0,535 Mol) Magnesiumspänen in 50 ml getrocknetem Tetrahydrofuran vorgelegt. Hierzu wurde eine Lösung von 93,5 g (0,50 Mol) 3-Bromanisol in 200 ml getrocknetem Tetrahydrofuran innerhalb von 90 Minuten so zugetropft, dass die Reaktionstemperatur 35° nicht überstieg. Nach erfolgter Zugabe wurde zur Vermeidung des Ausfallens des Grignardreagens mit zusätzlichen 150 ml getrocknetem Tetrahydrofuran verdünnt.

dab) In einem 1,5 l Vierhalskolben, versehen mit Rührer, Thermometer, Aufsatz für Inertgasbehandlung und $CO_2$/Aceton-Kühlbad, wurden 259,8 g (0,967 Mol) Diphenyl-chlorphosphat und 200 ml getrocknetes Tetrahydrofuran vorgelegt, und die Lösung auf -78° gekühlt. Dazu wurde nun innerhalb 2 Stunden die Lösung des gemäss baa) bereiteten Grignardreagens so zugetropft, dass die Reaktionstemperatur -70° nicht überstieg. Nach beendeter Zugabe liess man über Nacht unter Rühren auf Raumtemperatur erwärmen. Die Reaktionsmischung, welche etwas feinen weissen Niederschlag enthielt, wurde in ein 10 l Rührgefäss auf ein Gemisch von 2 l Eiswasser, 2 l ges. $NaHCO_3$-Lösung und 1 l Diäthyläther gegossen. Nach kräftigem Durchrühren während 10 Minuten wurde die wässrige Phase abgetrennt und die organische Phase nacheinander mit 1 l ges. $NaHCO_3$-Lösung, 200 ml 25% Ammoniak, 100 ml 25% Ammoniak und dreimal mit 500 ml ges. NaCl-Lösung gewaschen. Nach Trocknung über $MgSO_4$ wurde eingedampft, der Rückstand in 1 l Diäthyläther aufgenommen, und die Lösung bei 0° über Nacht stehengelassen. Der dabei ausgeschiedene weisse Festkörper (12 g) wurde durch Filtration entfernt und verworfen. Das Filtrat wurde eingedampft, am Hochvakuum (~ 10 Pa) getrocknet, der erhaltene Rückstand (163 g gelbes Oel) in 300 ml Hexan/Toluol 1:1 aufgenommen und die Lösung über 500 g Kieselgel filtriert. Durch Elution mit zuerst 3 l Hexan und 8 l Hexan/Essigsäureäthylester 9:1 und danach mit 2 l Hexan/Essigsäureäthylester 4:1 und 2 l Hexan/Essigsäureäthylester 7:3 erhielt man nach Trocknung im Hochvakuum (~ 10 Pa) während 1 Stunde bei 40° 118 g (3-Methoxyphenyl)phosphonsäure-diphenylester als leicht gelbliches Oel.

db) In einem 1,5 l Vierhalskolben, versehen mit Rührer, Thermometer, Aufsatz für Inertgasbehandlung, Tropftrichter mit Druckausgleich und $CO_2$/Aceton-Kühlbad, wurden 300 ml getrocknetes Tetrahydrofuran vorgelegt. Hierzu wurden 70 ml (0,412 Mol) 2,2,6,6-Tetramethylpiperidin mittels einer Spritze zugefügt, und die Lösung wurde auf -78° abgekühlt. In den Tropftrichter wurden via eine Stahikanüle 210 ml (0,336 Mol) 1,6N Butyllithium-Lösung in Hexan eingefüllt. Die Butyllithium-Lösung wurde innerhalb von ca. 10 Minuten in das Reaktionsgefäss eingetropft, wobei die Temperatur bis auf ca. -50° anstieg und ein weisser Niederschlag gebildet wurde. Das $CO_2$/Aceton-Kühlbad wurde durch ein Eis/Aethanol-Bad ersetzt, und die Reaktionsmischung wurde während 30 Minuten bei ca. -15° gerührt, dann wieder auf -78° gekühlt.

In einem separaten 1 l Rundkolben wurden unter Argon 250 ml getrocknetes Tetrahydrofuran und 95,2 g (0,280 Mol) (3-Methoxyphenyl)phosphonsäure-diphenylester (Material aus dab) vorgelegt, und die Lösung wurde auf -78° abgekühlt. Diese Lösung wurde nun via eine Stahlkanüle innerhalb von ca. 10 Minuten in die obige Reaktionsmischung einfliessen gelassen, wobei die Temperatur bis auf ca. -68° anstieg und eine durchscheinende, caramelfarbene Lösung entstand. Diese wurde noch 30 Minuten bei -78° gerührt.

In einem separaten 250 ml Schlenkrohr wurde unter Argon eine Lösung von 71,06 g (0,280 Mol) Jod in 150 ml getrocknetem Tetrahydrofuran hergestellt, und die Lösung wurde via Stahlkanüle in den Tropßrichter der Reaktionsapparatur transferiert. Die Reaktionsmischung wurde nun innerhalb 15 Minuten durch rasches Zutropfen der Jodlösung titriert, wobei die Reaktionstemperatur bis auf -65° anstieg. Nach Zutropfen von ca. 145 ml der ca. 170 ml Jodlösung, als eine rote Färbung der Reaktionsmischung bestehen blieb, wurde die Zugabe abgebrochen und man liess auf 0° erwärmen. Dann wurde die Reaktionsmischung mit 150 ml einer Lösung von 100 g Natriumthiosulfat-Pentahydrat in 200 ml entionisiertem Wasser versetzt, kräftig durchgerührt, und anschliessend mit 100 ml ges. $NaHCO_3$-Lösung versetzt. Das Zweiphasensystem wurde zur Entfernung von gebildetem Niederschlag filtriert und die Phasen getrennt. Die wässrige Phase wurde einmal mit 250 ml Essigsäureäthylester rückextrahiert, und die vereinigten organischen Phasen wurden mit 250 ml ges. NaCl-Lösung gewaschen, über $MgSO_4$ getrocknet und eingedampft. Der Rückstand wurde nochmals in 500 ml Essigsäureäthylester aufgenommen, und die Lösung wurde dreimal mit 250 ml entionisiertem Wasser und mit 250 ml ges. NaCl-Lösung gewaschen, über $MgSO_4$ getrocknet, filtriert und eingeengt. Der Rückstand (118 g gelbes Oel) wurde in 170 ml Toluol aufgenommen und die Lösung mit 115 ml Hexan versetzt, wobei ein weisser Niederschlag ausfiel. Dieser wurde durch Filtration entfernt und das Filtrat wurde auf eine Säule von 450 g Kieselgel aufgetragen. Zunächst wurden mit 2 l Hexan/Essigsäureäthylester 9:1 und 3 l Hexan/Essigsäureäthylester 8:2 Nebenprodukte eluiert. Anschliessend wurden mit 2 l Hexan/Essigsäureäthylester 7:3 die das Endprodukt enthaltenden Fraktionen eluiert. Nach Eindampfen und Trocknen am Hochvakuum (~ 10 Pa) während 1 Stunde bei 60° wurden

76,5 g eines orangen Oels erhalten. Dieses bestand gemäss $^{1}$H-NMR Analyse aus 75 Mol% (2-Jod-3-methoxyphenyl)phosphonsäure-diphenylester.

In zum Vorhergehenden analoger Weise, können die weiteren Liganden hergestellt werden.

## Patentansprüche

1. Verfahren zur Herstellung von Isoprenderivaten der allgemeinen Formel

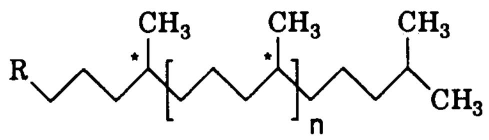

I

worin die Asymmetriezentren unabhängig voneinander die (R)- oder (S)-Konfiguration aufweisen können, R einen Rest der Formeln

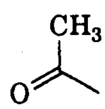

(a)

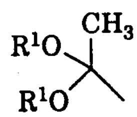

(b)

oder

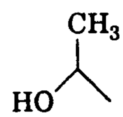

(c)

darstellt, $R^1$ niederes Alkyl ($C_1$-$C_4$) oder beide $R^1$ zusammen Aethylen oder Propylen bedeuten und n für die Zahl 0, 1 oder 2 steht, dadurch gekennzeichnet, dass man eine in der (E)- oder (Z)-Form vorliegende Verbindung der allgemeinen Formel

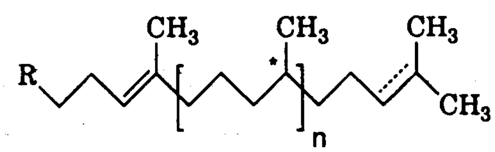

II

worin für R, n und ein gegebenenfalls vorhandenes Asymmetriezentrum obige Bedeutungen stehen, mit einem Ruthenium-Komplex eines optisch aktiven atropisomeren Diphosphins asymmetrisch hydriert.

2. Verfahren gemäss Anspruch 1, dadurch gekennzeichnet, dass man als Ruthenium-Komplex eines atropisomeren Diphosphins eine Verbindung der allgemeinen Formel verwendet:

$$[RuL]^{2+}(Z)_2\,, \qquad Ru(Z^1)_2L \qquad \text{oder} \qquad [Ru(Z^2)_{2-m}(L)(X)](Z^3)_m$$

III IV V

worin Z $BF_4^{\ominus}$, $ClO_4^{\ominus}$, $B(Phenyl)_4^{\ominus}$ oder $PF_6^{\ominus}$ darstellt, $Z^1$ Halogen oder die Gruppe Y-$COO^{\ominus}$ oder Y-$SO_3^{\ominus}$ bedeutet, Y niederes Alkyl ($C_1$-$C_4$), Phenyl, halogeniertes niederes Alkyl ($C_1$-$C_4$) oder halogeniertes Phenyl bedeutet, $Z^2$ Halogen, X Benzol, Hexamethylbenzol oder p-Cymol und m die Zahl 1 oder 2 darstellt, $Z^3$ Halogen, $BF_4^{\ominus}$, $ClO_4^{\ominus}$ oder $B(Phenyl)_4^{\ominus}$ bedeutet und L einen optisch aktiven, atropisomeren Diphosphin-Liganden bedeutet.

3. Verfahren gemäss Anspruch 1 oder 2, dadurch gekennzeichnet, dass man als atropisomeren Diphosphin-Liganden eine in (R)- oder (S)-Form vorliegende Verbindung verwendet der Formel

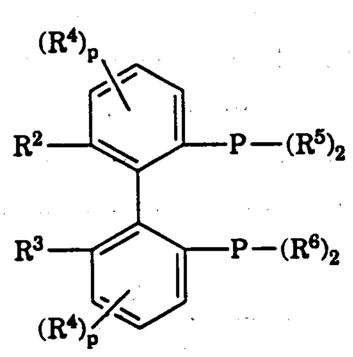

VI

worin $R^2$ und $R^3$ unabhängig voneinander niederes Alkyl ($C_1$-$C_4$), niederes Alkoxy ($C_1$-$C_4$), Hydroxy, geschütztes Hydroxy oder $R^2$ und $R^3$ zusammen -O-$CH_2$-O-$CH_2$-O- bedeuten, $R^4$ für niederes Alkyl ($C_1$-$C_4$) oder niederes Alkoxy ($C_1$-$C_4$) und p für die Zahl 0, 1 oder 2 stehen und $R^5$ und $R^6$ unabhängig voneinander Aryl, einen fünfgliedrigen Heteroaromaten oder zusammen mit dem Phosphoratom eine Gruppe der Formel

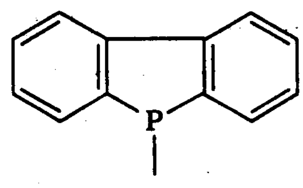

bedeuten.

4. Verfahren gemäss Anspruch 2 oder 3, dadurch gekennzeichnet, dass man als Ruthenium-Komplex eine Verbindung der allgemeinen Formel III verwendet.

**Claims**

1. A process for the manufacture of isoprene derivatives of the general formula

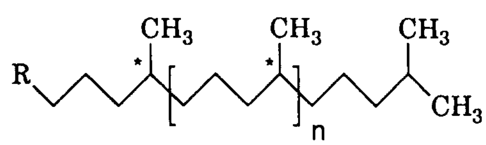

I

wherein the asymmetric centres each individually can have the (R) or (S) configuration, R represents a residue of the formula

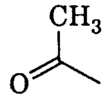

(a)

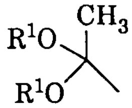

(b)

or

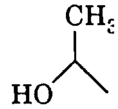

(c)

$R^1$ signifies lower alkyl ($C_1$-$C_4$) or both $R^1$'s together signify ethylene or propylene and n stands for the number 0, 1 or 2,
characterized by asymmetrically hydrogenating a compound, which is present in the (E) or (Z) form, of the general formula

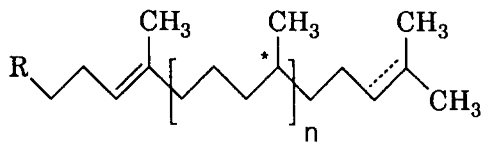

II

wherein R, n and an optionally present asymmetric centre have the above significances, with a ruthenium complex of an optically active atropisomeric diphosphine.

2. A process according to claim 1, characterized in that as the ruthenium complex of an atropisomeric diphosphine there is used of a compound of the general formula:

$$[RuL]^{2+}(Z)_2 \quad \text{III}, \qquad Ru(Z^1)_2L \quad \text{IV} \qquad \text{or} \qquad [Ru(Z^2)_{2-m}(L)(X)](Z^3)_m \quad \text{V}$$

wherein Z represents $BF_4^{\ominus}$, $ClO_4^{\ominus}$, $B(phenyl)_4^{\ominus}$ or $PF_6^{\ominus}$, $Z^1$ signifies halogen or the group Y-$COO^{\ominus}$ or Y-$SO_3^{\ominus}$, Y signifies lower alkyl ($C_1$-$C_4$), phenyl, halogenated lower alkyl ($C_1$-$C_4$) or halogenated phenyl, $Z^2$ represents halogen, X represents benzene, hexamethylbenzene or p-cymene and m represents the number 1 or 2, $Z^3$ signifies halogen, $BF_4^{\ominus}$, $ClO_4^{\ominus}$ or $B(phenyl)_4^{\ominus}$ and L signifies an optically active atropisomeric diphosphine ligand.

3. A process according to claim 1 or 2, characterized in that as the atropisomeric diphosphine ligand there is used a compound, which is present in the (R) or (S) form, of the formula

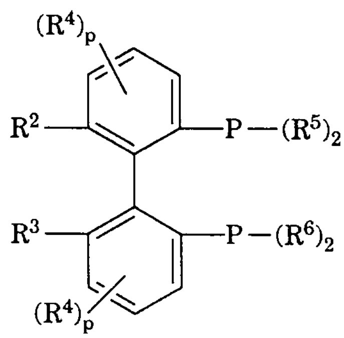

VI

wherein $R^2$ and $R^3$ each independently signify lower alkyl ($C_1$-$C_4$), lower alkoxy ($C_1$-$C_4$), hydroxy, protected hydroxy or $R^2$ and $R^3$ together signify -O-$CH_2$-O-$CH_2$-O-, $R^4$ stands for lower alkyl ($C_1$-$C_4$) or lower alkoxy ($C_1$-$C_4$) and p stands for the number 0, 1 or 2 and $R^5$ and $R^6$ each independently signify aryl, a five-membered heteroaromatic or together with the phosphorus atom a group of the formula

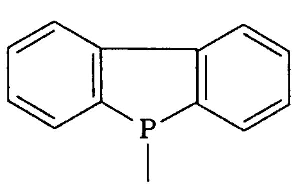

4. A process according to claim 2 or 3, characterized in that a compound of general formula III is used as the ruthenium complex.

**Revendications**

1. Procédé pour préparer des dérivés de l'isoprène de formule générale

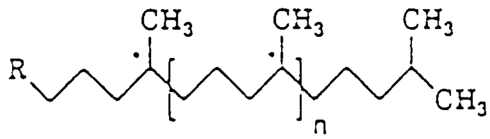

I

où les centres d'asymétrie indépendants les uns des autres peuvent présenter la configuration (R) ou (S), R est un radical des formules

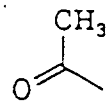

(a)

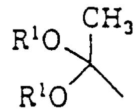

(b)

ou

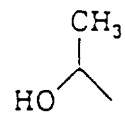

(c)

$R^1$ représente un alkyle inférieur en $C_1$-$C_4$, ou les deux $R^1$ ensemble représentent l'éthylène ou le propylène et n représente le nombre 0, 1 ou 2, caractérisé en ce que l'on hydrogène de façon asymétrique un composé qui se présente dans la forme (E) ou (Z), de formule générale

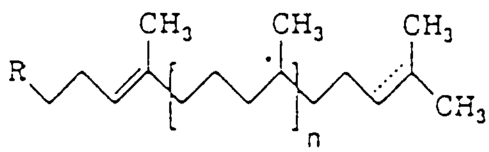

II

où R, n, et un centre d'asymétrie éventuellement présent, ont les significations précédentes,
avec un complexe du ruthénium d'une diphosphine optiquement active, atropo-isomère actif.

2. Procédé selon la revendication 1, caractérisé en ce que l'on utilise comme complexe du ruthénium d'une diphosphine atropo-isomère un composé de formule générale :

$$[RuL]^{2+}(Z)_2 \quad \text{III}, \qquad Ru(Z^1)_2L \quad \text{IV} \qquad \text{ou} \qquad [Ru(Z^2)_{2-m}(L)(X)](Z^3)_m \quad \text{V}$$

où Z représente $BF_4^-$, $ClO_4^-$, B(phényl)$_4^-$ou $PF_6^-$, $Z^1$ représente un halogène ou le groupe Y-COO$^-$ou Y-$SO_3^-$, Y représente un alkyle inférieur en $C_1$-$C_4$, le phényle, un alkyle inférieur en $C_1$-$C_4$ halogéné ou un phényle halogéné, $Z^2$ représente un halogène, X représente le benzène, l'hexaméthylbenzène ou le p-cymol et m représente le nombre 1 ou 2, $Z^3$ représente un halogène, $BF_4^-$, $ClO_4^-$ou B(phényl)$_4^-$et L représente un ligand diphosphine atropo-isomère, optiquement actif.

3. Procédé selon la revendication 1 ou 2, caractérisé en ce que comme ligand diphosphine atropo-isomère, on utilise un composé qui se présente sous la forme (R) ou (S), de formule

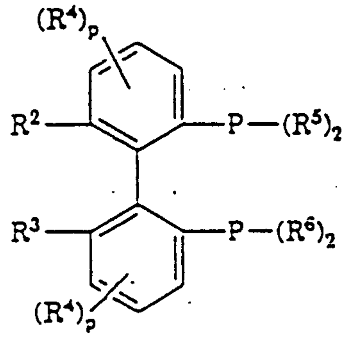

VI

où $R^2$ et $R^3$, indépendamment l'un de l'autre, représentent un alkyle inférieur en $C_1$-$C_4$, un alcoxy inférieur en $C_1$-$C_4$, l'hydroxy, un hydroxy protégé ou $R^2$ et $R^3$ représentent ensemble $-O-CH_2-O-CH_2-O-$, $R^4$ représente un alkyle inférieur en $C_1$-$C_4$, ou un alcoxy inférieur en $C_1$-$C_4$, et p représente le nombre 0, 1 ou 2, et $R^5$ et $R^6$ indépendamment l'un de l'autre représentent un aryle, un hétéroaromatique à 5 chaînons, ou, avec l'atome de phosphore, ils représentent un groupe de formule

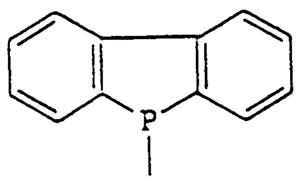

4. Procédé selon la revendication 2 ou 3, caractérisé en ce que l'on utilise comme complexe de ruthénium un composé de formule générale III.